# EUROPEAN PATENT SPECIFICATION

(11) **EP 2 260 765 B1**
(45) Date of publication and mention of the grant of the patent: **06.03.2013**
(21) Application number: 10165412.7
(22) Date of filing: 09.06.2010
(51) Int. Cl.: A61B 8/10, A61B 3/16, A61B 3/103, A61B 3/107

(54) **Non-contact ultrasonic measurement device**
Kontaktfreie Messvorrichtung
Dispositif de mesure ultrasonique sans contact

(30) Priority: 09.06.2009 JP 2009138620
(43) Date of publication of application: 15.12.2010
(73) Proprietor: NIDEK CO., LTD, Gamagori-shi Aichi 443-0038 (JP); MicroAcoustic Instruments Inc., Gatineau, QC J9H 1A8 (CA)
(72) Inventor: Miwa, Tetsuyuki, Gamagori-shi Aichi 443-0038 (JP); Jinde, Masayuki, Gamagori-shi Aichi 443-0038 (JP); Schindel, David W., Gatineau, Quebec J9H 1A8 (CA)
(74) Representative: Prüfer & Partner GbR European Patent Attorneys

(56) References cited:
- EP-A1- 2 113 191
- US-A- 5 325 135
- US-A- 5 865 742

## Description

### Technical Field

The present invention relates to a non-contact ultrasound measurement device for measuring characteristics of an object to be examined non-contactively by use of ultrasonic waves.

### Background Art

As one of non-contact ultrasound measurement devices, there is recently proposed a non-contact ultrasound tonometer including a transducer having a vibrator for emitting an ultrasonic wave toward a cornea of an examinee's eye and a sensor for detecting the ultrasonic wave reflected by the cornea to measure intraocular pressure of the eye in a non-contact manner (see Patent Literature 1).

US 5 865 742 A (Patent Literature 2) forms the closest prior art document. It describes how a beam of acoustic energy comprising acoustic radiation and the associated acoustic streaming of an ultrasonic beam is used to indent or applanate (flatten) the eye. These two effects produce acoustic pressure on the eye which partially overcomes the IOP of the eye. Such concurrent determination of acoustic pressure and distortion of the eye is then used to determine the IOP.

### Citation List

### Patent Literature

Patent Literature 1: WO 2008/072527

Patent Literature 2 : US 5 865 135 A.

### Summary of Invention

### Technical Problem

Meanwhile, the ultrasound measurement device mentioned above is preferably arranged to enable good observation of the object located in a position away from the device. The ultrasound measurement device is further preferably arranged to enable good measurement of various data on the object.

For instance, the device in Patent Literature 1 is designed to merely measure a schematic eye. Thus such device is still insufficient to measure a real human eye and has many problems to be solved for practical use. To mitigate fear of an examinee, for example, it is necessary a certain degree of a working distance (e.g., about 10 mm) between the cornea and the device. It is also preferable to satisfactorily observe a state of the eye held in a position away from the device. Furthermore, this device could not measure second eye characteristic different from the intraocular pressure.

The present invention has a purpose to provide a non-contact ultrasound measurement device capable of accurately measuring a first characteristic of an object to be examined and appropriately observing the object or measuring a second characteristic of the object.

### Solution to Problem

To achieve the above purposed, the present invention provides a non-contact ultrasound measurement device according to claim 1.
Further developments of the present invention are given in the dependent claims.

### Advantageous Effects of Invention

According to the invention, it is possible to accurately measure a first characteristic of an object to be examined and appropriately observe the object or measure a second characteristic of the object.

### Brief Description of Drawings

FIG. 1 is a schematic configuration view of a measurement system and an optical system of a non-contact ultrasound tonometer which is a non-contact ultrasound measurement device in an embodiment;
FIG. 2 is a schematic configuration view of a transducer seen from front in the embodiment;
FIG. 3 is a schematic block diagram of a control system of the device in the embodiment.
FIGs. 4A and 4B are views showing modified examples of the shape of a cross section of a transmitting and receiving part in the embodiment;
FIG. 5 is a schematic configuration view of a measurement system and an optical system in a concrete example in which an measuring optical system is provided in addition to an intraocular pressure measuring system; and
FIG. 6 is a view showing an example of a configuration in which an aperture of the transducer is used for measurement of a second characteristic of an object to be examined.

### Description of Embodiments

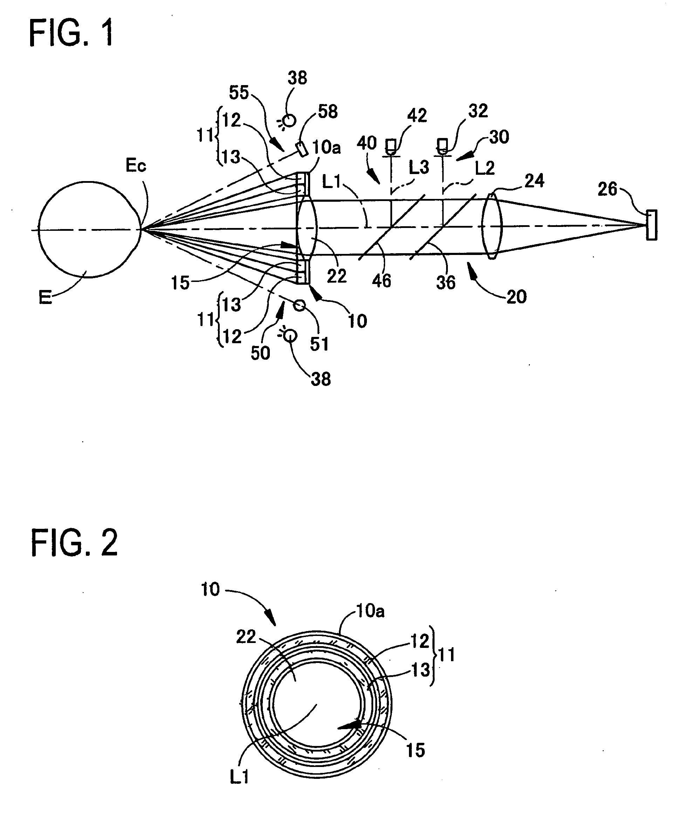

A detailed description of a preferred embodiment of the present invention will now be given referring to the accompanying drawings. FIG. 1 is a schematic configuration view of a measurement system and an optical system of a non-contact ultrasound tonometer which is a non-contact ultrasound measurement device in this embodiment. The following measurement system and optical system are built in a housing not shown. This housing may be moved three-dimensionally relative to an examinee's eye by a known movement mechanism for alignment or may be a handheld type (handy type).

An ultrasound transducer 10 emits an ultrasonic wave (a pulse wave or a continuous wave) toward a cornea Ec of the examinee's eye E by the medium of air and also detects the ultrasonic wave reflected by the cornea Ec. The transducer 10 includes, as a transmitting and receiving part 11 of ultrasonic wave, a transmitting section 12 for emitting an ultrasonic wave and a receiving section 13 for detecting a reflection wave from the cornea Ec. The transducer 10 is used for measuring intraocular pressure in a non-contact manner. In the transducer 10, the transmitting section 12 and the receiving section 13 are separately configured and placed in different positions, but they are not limited to such configuration and may be shared.

The optical system of the device includes an observation optical system 20 for observing an anterior segment of the eye E from front, a fixation target projecting (presenting) optical system 30 for inducing the eye E to hold fixation, a first target projecting optical system 40 for projecting a first target onto the cornea Ec to detect an alignment state in up-down and right-left directions, a second target projecting optical system 50 for projecting a second target onto the cornea Ec to detect an alignment state in a working distance which is a front-rear direction, and a target detecting optical system 55 for detecting the second target projected onto the cornea Ec.

The observation optical system 20 includes an objective lens 22, an image forming lens 24, and a two-dimensionally imaging element 26. This optical system 20 is placed so that its optical axis (an observation optical axis) L1 is almost coaxial with a central axis of the transmitting and receiving part 11. Accordingly, when the observation optical axis L1 is aligned with respect to a predetermined portion (e.g., the center of a cornea, the center of a pupil) of the eye E, the transducer 10 is aligned in up-down and right-left directions relative to the eye E.

An image of the anterior segment illuminated by an infrared light source 38 passes through an aperture 15, the objective lens 22, a half mirror 46, and a dichroic mirror 36 in order and then forms an image on the imaging element 26 through the image forming lens 24. The anterior segment image imaged by the imaging element 26 is displayed on a monitor 72 mentioned later.

The fixation target projecting optical system 30 includes a visible light source 32 to project a fixation target onto the eye E to induce the eye E to hold fixation. Visible light from the light source 32 is reflected by the dichroic mirror 36 that transmits infrared light but reflects visible light. This light then passes through the half mirror 46 and the objective lens 22 and is projected onto a fundus of the eye E. An optical axis L2 of the fixation target projecting optical system 30 is made coaxial with the observation optical axis L1 by the dichroic mirror 36 placed in the optical path (the observation optical path) of the observation optical system 20.

The first target projecting optical system 40 includes an infrared light source 42 and projects infrared light onto the cornea Ec from front so that the infrared light forms the first target for detecting the alignment state in the up-down and right-left directions. The infrared light from the light source 42 is reflected by the half mirror 46, passes through the objective lens 22, and is projected onto the cornea Ec. An optical axis L3 of the first target projecting optical system 40 is made coaxial with the observation optical axis L1 by the half mirror 46 placed in the observation optical path (the optical path of the observation light).

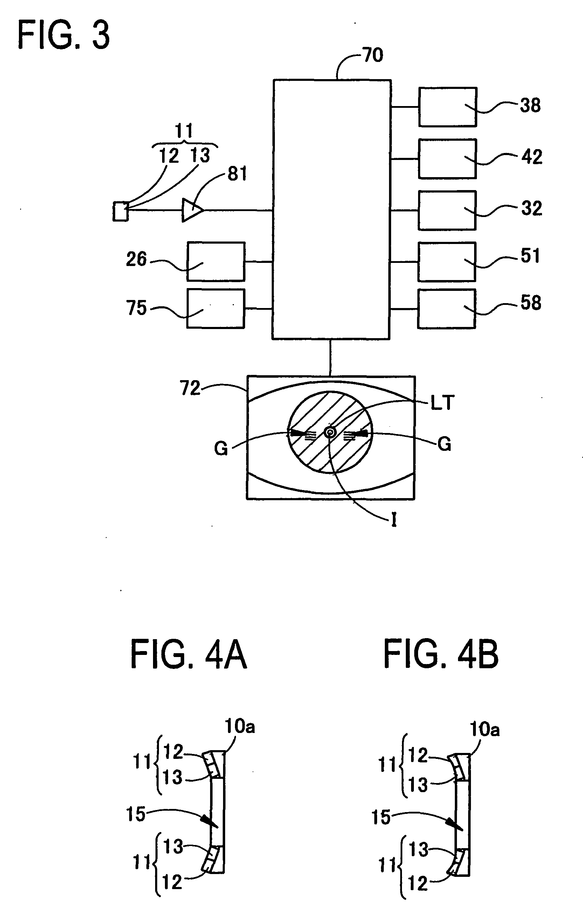

The observation optical system 20 detects the first target formed on the cornea Ec (i.e., the optical system 20 receives the infrared light that is emitted from the light source 42 and reflected by the cornea Ec) by the first target projecting optical system 40. In other words, the observation optical system 20 is also used as a target detecting optical system. The first target image imaged by the imaging element 26 is displayed on the monitor 72 (see a point image I in FIG. 3).

The second target projecting optical system 50 includes an infrared light source 51 and obliquely projects infrared light onto the cornea Ec so that the infrared light forms a second target for detecting an alignment state in the working distance direction.

The target detecting optical system 55 includes a position detecting device (e.g., a line CCD) 58 and detects the second target image formed on the cornea Ec by the second target projecting optical system 50 (i.e., the optical system 55 receives the infrared light that is emitted from the light source 51 and reflected by the cornea Ec). It is to be noted that detection of the alignment state in the working distance direction may be conducted by the transducer 10. For example, the time until an ultrasonic wave emitted to the examinee's eye returns to the transducer 10 is converted into distance.

FIG. 2 is a schematic configuration view of the transducer 10 seen from front in the present embodiment. The transducer 10 centrally includes the aperture 15 having a sufficient size to be used as an observation optical path and the transmitting and receiving part 11 is placed to surround the aperture 15.

To be more specific, the base part 10a is formed with a through hole having an inside diameter corresponding to the aperture 15, and the transmitting and receiving part 11 is placed on the base part 10a on a side facing the eye E and in an almost annular form outside the through hole. The transmitting section 12 and the receiving section 13 are placed concentrically in different positions.

In FIG. 2, the transmitting section 12 is located outside and the receiving section 13 is located inside. Alternatively, the receiving section 13 may be placed outside and the transmitting section 12 may be inside.

The objective lens 22 is placed in the aperture 15. It is to be understood that other optical member(s) (e.g., a glass plate, a filter, etc.) may be placed in the aperture 15 or the optical member as mentioned above may not be placed in the aperture 15.

The aperture 15 has a size enough for observation of the examinee's eye by the observation optical system 20 to ensure an observation range in a permissible range by the observation optical system 20. For instance, the size of the aperture 15 (the inside diameter of the through hole of the base part 10a) is appropriately determined by considering whether or not alignment with respect to the examinee's eye is smoothly performed, whether or not a state of the anterior segment is observed well, or others. The shape of the aperture 15 is not limited to a perfect circle and may be any other shape (e.g., rectangular, elliptic, and others).

The transmitting and receiving part 11 is preferably an air-coupled ultrasound transmitting and receiving part (an ultrasound transducer) for transmitting and receiving an ultrasonic wave having a broadband frequency component in order to enhance propagation efficiency in air. In this case, a BAT™ transducer manufactured by MicroAcoustic Instrument Inc. is available. For the details of such transducer, see US 5,287,331 and JP-A-2005-506783, for example. It is to be understood that the transducer is not limited thereto and may be a piezoelectric ultrasound transmitting and receiving part (ultrasound transducer).

FIG. 3 is a schematic block diagram of a control system of the device in the present embodiment. An arithmetic controller 70 performs control of the entire device. This controller 70 processes an output signal from the transducer 10 and determines intraocular pressure of the eye E. The transducer 10 (the transmitting and receiving part 11) is connected to the amplifier 81. Thus, an electric signal output from the transducer 10 is amplified by the amplifier 81 and transmitted to the controller 70. The controller 70 is also connected to the imaging element 26, the light sources 32, 38, 42, and 52, the position detecting device 58, the monitor 72, the memory 75, and others. The memory 75 stores in advance a measurement program for measuring the intraocular pressure by use of the transducer 10, a control program for controlling the entire device, and other programs.

The case of measuring the intraocular pressure of the eye E by the device having the above configuration is explained below. An examiner firstly induces an examinee to look at a fixation target. While observing the anterior segment image appearing on the monitor 72, the examiner makes alignment of the transducer 10 with respect to the eye E. At that time, the arithmetic controller 70 displays the anterior segment image imaged by the imaging element 26 including a first target image I, and a reticule LT and an indicator G mentioned later, on the monitor 71 as shown in FIG. 3. The controller 70 detects an alignment state in the working distance direction based on an output signal from the position detecting device 58 and controls the display of the indicator G based on the detection result.

The examiner makes alignment of the device in the up-down and right-left directions to bring the first target image I into the reticule LT. The examiner further makes alignment in the working distance direction so that the indicator G appears in an appropriate display state (for example, the indicator appears as a single line).

After completion of alignment in the up-down, right-left, and front-back directions, when a predetermined trigger signal is manually or automatically input, the arithmetic controller 70 causes the transmitting section 12 to emit the ultrasonic wave toward the center of the cornea Ec and causes the receiving section 13 to detect the ultrasonic wave reflected by the cornea Ec. The controller 70 determines the intraocular pressure of the eye E based on the detection result by the transmitting and receiving part 11 and displays the result on the monitor 72. Specifically, the controller 70 obtains an intraocular pressure value based on a relationship between amplitude of the ultrasonic wave detected by the transmitting and receiving part 11 and intraocular pressure. For instance, the intraocular pressure is calculated by utilizing the tendency that an amplitude level of the reflection wave is larger as the intraocular pressure value is larger.

As described above, the transmitting and receiving part 11 is placed outside the observation optical path and hence the areas of the transmitting section 12 and the receiving section 13 are ensured and the observation optical path is not interrupted. Since the transmitting and receiving part 11 is placed to surround the aperture 15, it is possible to increase an S/N ratio of a detection signal of the reflection wave from the cornea Ec detected by the transducer 10 even when the working distance from the eye E is increased.

In other words, the region for observation of the anterior segment is appropriately placed in the central portion and the region for transmission and reception of the ultrasonic wave is appropriately placed in a peripheral portion relative to the cornea. Accordingly, the transducer 10 and the observation optical system 20 are optimized to enable appropriate measurement of intraocular pressure and observation of the anterior segment by transmission and reception of the ultrasonic wave.

Since the transmitting and receiving part 11 (the transmitting section 12 and the receiving section 13) is designed to be almost annular, an incident wave to the cornea Ec is almost uniform and thus the reflection wave from the cornea Ec can be precisely detected.

A cross-sectional shape of the transmitting and receiving part 11 on the eye E side is not limited to the flat shape as shown in FIG. 1 but may be conical (or tapered) as shown in FIG. 4A or spherical as shown in FIG. 4B.

The transmitting section 12 and the receiving section 13 are not limited to the double ring structure shown in FIG. 2 but may be configured as a triple or more ring structure. In other words, at least one of the transmitting section 12 and the receiving section 13 may be configured as a double or more ring structure.

The transmitting and receiving part 11 is not limited to a configuration surrounding almost the entire aperture 15 as shown in FIG. 2 but may be designed to surround a part of the circumstance of the aperture 15. In the case where the transmitting and receiving part 11 is placed in almost annular form, accordingly, it may be arranged in an intermittent circular form instead of a continuous circular form.

The shape of the transmitting and receiving part 11 is preferably almost annular but may be variously modified. For instance, it may be polygonal (a polygon having the larger number of sides is more preferable).

In addition to the configuration for measuring the intraocular pressure with the ultrasonic wave by use of the transducer 10 (the transmitting and receiving part 11), a measuring optical system may be provided to measure a second characteristic of the eye E different from the intraocular pressure. In this case, the aperture 15 is used as a measuring optical path (an optical path of measurement light). The aperture 15 is also designed to have a sufficient size to measure the examinee's eye by the measurement optical system in order to ensure permissible measurement precision. The measurement optical system may include for example an eye refractive power measurement optical system adapted to project measurement light onto the fundus of the eye E and receive reflection light therefrom to measure eye refractive power, a corneal shape measurement optical system adapted to project measurement light onto the cornea Ec and receive reflection light therefrom to measure a corneal shape, an eye axial length measurement optical system adapted to receive interference light by measurement light and reference light, and others to measure an axial length.

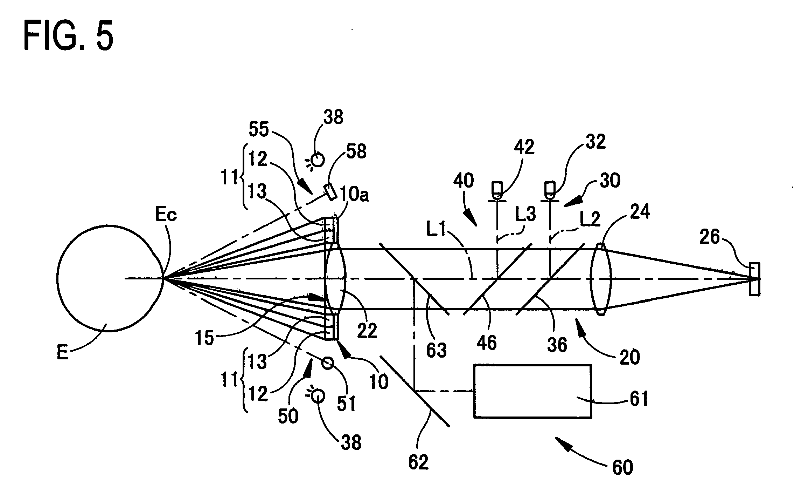

FIG. 5 is a schematic configuration view of a measurement system and an optical system in a concrete example in the case where the measurement optical system is provided in addition to the intraocular pressure measurement system. The components assigned with the same reference signs as those in FIG. 1 have the same functions and configurations as those in FIG. 1 unless particular explanation. The intraocular pressure measurement is performed by the same configuration as mentioned above and thus the explanation thereof is not repeated here.

A measurement optical system 60 includes a measurement optical unit 61 having a structure to project measurement light onto the eye E and receive reflection light therefrom, a total reflection mirror 62, a beam splitter 63 (e.g., a half mirror and a dichroic mirror) that transmits anterior segment observation light and reflects measurement light, and an objective lens 22. In the case where the eye refractive power measurement unit is provided as the measurement optical unit 61, the fixation target projecting optical system 30 is preferably a known structure capable of fogging a fixation target.

Measurement light emitted from a measurement light source of the measurement optical unit 61 is reflected by the mirror 62 and the beam splitter 63, then passes through the objective lens 22 and enters the eye E. The measurement light reflected by a predetermined portion of the eye E such as the fundus, the cornea, and other passes through the objective lens 22, is reflected by the beam splitter 63 and the mirror 62, and received by a light receiving element, an imaging element, and others in the measurement optical unit 61. The arithmetic controller 70 performs a test (e.g., measurement of eye characteristics, acquisition of an image of an examinee's eye, etc.) on the eye E based on a light receiving result (an imaging result).

With such configuration, a complex type ophthalmic device including the intraocular pressure measurement system and the measurement optical system can be made very compact as compared with a conventional device.

The device is not limited to the above configuration. For example, a projecting optical system for projecting a target onto the cornea Ec to measure corneal curvature (e.g., a ring target and a Placido target) is further provided so that a target image projected on the cornea Ec is imaged by the observation optical system 20 (the imaging element 26) to measure the corneal curvature. In this case, the target projecting optical system for measuring the corneal curvature is placed without interfering with the transmitting and receiving part 11.

Furthermore, the device may additionally include a light projecting optical system adapted to project slit light onto an anterior segment of the eye E through the aperture 15 and an imaging optical system having an imaging optical axis intersecting with the observation optical axis (the measurement optical axis) L1 at a predetermined angle and being adapted to receive reflection light from the anterior segment and image a cross-sectional image of the anterior segment, in order to measure corneal thickness, anterior chamber depth, and others based on the cross-sectional image of the imaged anterior segment. In this case, the imaging optical system is placed without interfering with the transmitting and receiving part 11.

As another alternative, the device may include an illumination optical system having an illumination optical axis intersecting with the observation optical axis L1 at a predetermined angle and being adapted to obliquely illuminate illumination light to the cornea Ec and an imaging optical system having an imaging optical axis intersecting with the observation optical axis L1 at a predetermined angle and being adapted to receive reflection light from the cornea Ec and image a corneal endothelial cell image, in order to image the corneal endothelial cell image. In this case, the illumination optical system and the imaging optical system are placed without interfering with the transmitting and receiving part 11.

The present invention is not limited to the configuration for measuring intraocular pressure by use of the transducer 10 and may be applied to any configuration for measuring internal pressure of an object to be examined (not limited to a living body) by use of the transducer 10. Furthermore, the invention may be applied to any configuration for measuring the characteristics of the object (e.g., stress distribution, shape) by use of the transducer 10.

In the above case, the device is provided with the observation unit for observing the object from front in a non-contact manner (e.g., an optical observation unit, an X-ray observation unit, an ultrasound observation unit, and an acoustic observation unit) and the transducer (a first unit) including the aperture having a sufficient size for observation of the object by the observation unit (see FIG. 1). In this case, the object is observed with the observation unit through the aperture 15 of the transducer 10. Preferably, the central axis of the observation unit and the central axis of the transmitting and receiving part 11 are placed in an almost coaxial relation.

In the above explanation, the aperture 15 of the transducer 10 is used to measure the second characteristic of the eye E. The invention may also be applied to any configuration utilizing the aperture 15 of the transducer 10 to measure the second characteristic of the object.

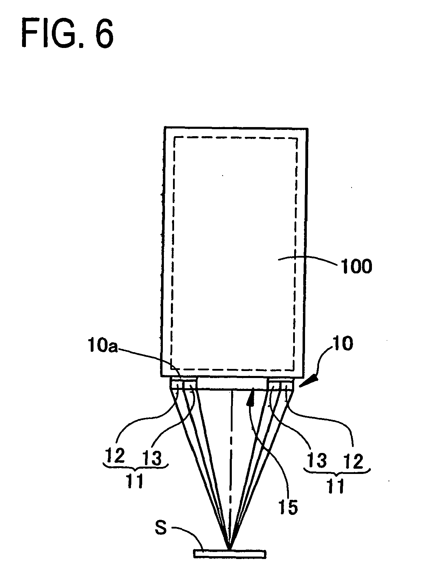

In the above case, the device includes a second measurement unit 100 (e.g., an optical measurement unit, an X-ray measurement unit, an ultrasound measurement unit, an acoustic measurement unit) for measuring the second characteristic of an object S from front in a non-contact manner, and the transducer (the first unit) including the aperture 15 having a sufficient size for measuring the object S by the measurement unit 100 (see FIG. 6). In this case, the second characteristic of the object S is measured by the measurement unit 100 through the aperture 15 of the transducer 10. The central axis of the measurement unit 100 and the central axis of the transmitting and receiving part 11 are preferably placed in an almost coaxial relation.

Specifically, the measurement unit 100 emits and/or detects a measurement wave to measure the second characteristic of the object S. The measurement wave may be selected according to measurement purposes from an electromagnetic wave (electric wave, light, X-rays, gamma rays, etc.), a sonic wave (ultrasonic wave, acoustic energy), and others.

With the above structure, a first ultrasound measurement system including the transducer having the aperture and the second measurement system are separately arranged and respective measurements are optimized.

The present invention is not limited to a configuration to be used in air but may be applied to any configuration to be used in gases. For instance, it is conceived that the invention is used in gases containing air under atmosphere pressure or in any gases (including inert gas) under arbitrary pressure.

While the presently preferred embodiment of the present invention has been shown and described, it is to be understood that this disclosure is for the purpose of illumination and that various changes and modifications may be made without departing from the scope of the invention as set forth in the appended claims.

### Reference Signs List

- 10: Ultrasound transducer
- 11: Transmitting and receiving part
- 12: Transmitting section
- 13: Receiving section
- 15: Aperture
- 20: Observation optical system
- 50: Second target projecting optical system
- 55: Target detecting optical system
- 60: Measurement optical system
- 100: Second measurement unit

## Claims

1. A non-contact ultrasound measurement device comprising:
a first unit (10, 70) including an ultrasound transducer (10) adapted to transmit and receive an ultrasonic wave in a non-contact manner with respect to an object to be examined (E, S), the first unit being adapted to process an output signal from the transducer and measure a first characteristic of the object, the first characteristic being internal pressure of the object; and
a second unit (20, 60, 70, 100) including at least one of an observation unit (20) for observing the object from front in a non-contact manner and a measurement unit (60, 100) for measuring a second characteristic of the object from front in a non-contact manner,
wherein the transducer comprises:
an aperture (15) being provided in the central part of the transducer and having a sufficient size to be utilized for observation or measurement of the object by the second unit, and
a transmitting and receiving part (11) of the ultrasonic wave being provided to surround the aperture,
wherein
the transmitting and receiving part (11) includes a transmitting section (12) for emitting an ultrasonic beam toward the object, and a receiving section (13) for detecting the ultrasonic beam reflected by the object, and
characterized in that the first unit is configured to obtain internal pressure of the object based on a relationship between the amplitude of the ultrasonic beam detected by the receiving section and the internal pressure of the object.

2. The non-contact ultrasound measurement device according to claim 1, wherein the observation unit or the measurement unit includes at least one of an optical unit, an X-ray unit, an ultrasound unit, and an acoustic unit.

3. The non-contact ultrasound measurement device according to one of claims 1 to 2, wherein the observation unit includes an objective lens (22) to be placed before the object, and the objective lens is placed in the aperture.

4. The non-contact ultrasound measurement device according to one of claims 1 to 3, wherein the ultrasound transducer is a broadband air-coupled transducer for transmitting and receiving the ultrasonic wave having a broad band frequency component.

5. The non-contact ultrasound measurement device according to one of claims 1 to 4, wherein the transmitting and receiving part is placed in an almost annular form outside the aperture.

6. The non-contact ultrasound measurement device according to one of claims 1 to 5, wherein a transmitting section and a receiving section of the transmitting and receiving part are arranged concentrically in different positions.

7. The non-contact ultrasound measurement device according to one of claims 1 to 6, wherein
the object is an examinee's eye,
the first characteristic is intraocular pressure which is a first eye characteristic of the examinee's eye, and
the observation unit is an observation optical system adapted to observe an anterior segment of the examinee's eye from front.

8. The non-contact ultrasound measurement device according to claim 7, wherein
the transmitting section is adapted to transmit an ultrasound pulse wave toward the center of a cornea of the examinee's eye and the receiving section is adapted to receive the ultrasound pulse wave reflected by the center of the cornea, and
the first measurement unit obtains an intraocular pressure value based on a relationship between wave characteristics of the reflected ultrasound pulse wave detected by the receiving section and the intraocular pressure.

9. The non-contact ultrasound measurement device according to claim 7 or 8, wherein
the measurement unit is a measurement optical system adapted to measure a second eye characteristic of the examinee's eye as the second characteristic from front, and the aperture surrounded by the transmitting and receiving part is used as a measurement optical path of the measurement optical system.

10. The non-contact ultrasound measurement device according to claim 9, wherein the measurement optical system includes at least one of an eye refractive power measurement optical system adapted to project measurement light onto a fundus of the examinee's eye and receive reflection light from the fundus to measure eye refractive power of the examinee's eye as the second eye characteristic and a corneal shape measurement optical system adapted to project measurement light onto a cornea of the examinee's eye and receive reflection light from the cornea to measure a shape of the cornea as the second eye characteristic.

## Patentansprüche

1. Kontaktfreie Ultraschallmessvorrichtung, aufweisend:
eine erste Einheit (10, 70), die einen Ultraschalltransducer (10) enthält, der daran angepasst ist, eine Ultraschallwelle auf kontaktfreie Weise bezüglich einem zu untersuchenden Objekt (E, S) zu übertragen und zu empfangen, wobei die erste Einheit daran angepasst ist, ein Ausgabesignal von dem Transducer zu verarbeiten und eine erste Eigenschaft des Objekts zu messen, wobei die erste Eigenschaft ein Innendruck des Objekts ist; und
eine zweite Einheit (20, 60, 70, 100), die wenigstens entweder eine Beobachtungseinheit (20) zum Beobachten des Objekts von vorne auf kontaktfreie Weise oder eine Messeinheit (60, 100) zum Messen einer zweiten Eigenschaft des Objekts von vorne auf eine kontaktfreie Weise enthält,
wobei der Transducer aufweist:
eine Apertur (15), die in einem zentralen Teil des Transducers vorgesehen ist und eine ausreichende Größe hat, um für Beobachtung oder Messung des Objekts durch die zweite Einheit verwendet zu werden, und
einen Übertragungs- und Empfangsteil (11) der Ultraschallwelle, der die Apertur umgebend vorgesehen ist,
wobei
der Übertragungs- und Empfangsteil (11) einen Übertragungsabschnitt (12) zum Aussenden eines Ultraschallstrahls zu dem Objekt und einen Empfangsabschnitt (13) zum Erfassen des Ultraschallstrahls, der von dem Objekt reflektiert wird, enthält, und
dadurch gekennzeichnet, dass die erste Einheit so konfiguriert ist, dass ein Innendruck des Objekts basierend auf einer Beziehung zwischen der Amplitude des Ultraschallstrahls, der von dem Empfangsabschnitt erfasst wird, und dem Innendruck des Objekts erhalten wird.

2. Kontaktfreie Ultraschallmessvorrichtung gemäß Anspruch 1, wobei die Beobachtungseinheit oder die Messeinheit wenigstens entweder eine optische Einheit, eine Röntgeneinheit, eine Ultraschalleinheit oder eine akustische Einheit enthält.

3. Kontaktfreie Ultraschallmessvorrichtung gemäß einem der Ansprüche 1 bis 2, wobei die Beobachtungseinheit eine Objektivlinse (22) enthält, die vor dem Objekt zu platzieren ist, und die Objektivlinse in der Apertur platziert ist.

4. Kontaktfreie Ultraschallmessvorrichtung gemäß einem der Ansprüche 1 bis 3, wobei der Ultraschalltransducer ein Breitband-Luftgekoppelter Transducer zum Übertragen und Empfangen der Ultraschallwelle ist, die eine Breitbandfrequenzkomponente hat.

5. Kontaktfreie Ultraschallmessvorrichtung gemäß einem der Ansprüche 1 bis 4, wobei der Übertragungs- und Empfangsteil nahezu ringförmig außerhalb der Apertur angeordnet ist.

6. Kontaktfreie Ultraschallmessvorrichtung gemäß einem der Ansprüche 1 bis 5, wobei ein Übertragungsabschnitt und ein Empfangsabschnitt des Übertragungs- und Empfangsteils konzentrisch in verschiedenen Positionen angeordnet sind.

7. Kontaktfreie Ultraschallmessvorrichtung gemäß einem der Ansprüche 1 bis 6, wobei
das Objekt ein Auge eines Prüflings ist,
die erste Eigenschaft ein Augeninnendruck ist, der eine erste Augeneigenschaft des Auges des Prüflings ist, und
die Beobachtungseinheit ein optisches Beobachtungssystem ist, das daran angepasst ist, ein vorderes Segment des Auges des Prüflings von vorne zu beobachten.

8. Kontaktfreie Ultraschallmessvorrichtung gemäß Anspruch 7, wobei
der Übertragungsabschnitt daran angepasst ist, eine Ultraschallpulswelle zu dem Zentrum einer Hornhaut des Auges des Prüflings zu übertragen und der Empfangsabschnitt daran angepasst ist, die Ultraschallpulswelle, die von dem Zentrum der Hornhaut reflektiert wird, zu empfangen, und
die erste Messeinheit einen Augeninnendruckwert erhält, der auf einer Beziehung zwischen Welleneigenschaften der reflektierten Ultraschallpulswelle, der von dem Empfangsabschnitt erfasst wird, und dem Augeninnendruck basiert.

9. Kontaktfreie Ultraschallmessvorrichtung gemäß Anspruch 7 oder 8, wobei
die Messeinheit ein optisches Messsystem ist, das daran angepasst ist, eine zweite Augeneigenschaft des Auges des Prüflings als die zweite Eigenschaft von vorne zu messen, und die Apertur, die von dem Übertragungs- und Empfangsteil umgeben ist, als ein optischer Messpfad des optischen Messsystems verwendet ist.

10. Kontaktfreie Ultraschallmessvorrichtung gemäß Anspruch 9, wobei das optische Messsystem zumindest entweder ein optisches Augenbrechkraftmesssystem ist, das daran angepasst ist, Messlicht auf einen Hintergrund des Auges des Prüflings zu projizieren und Reflexionslicht von dem Hintergrund zu empfangen, um eine Augenbrechkraft des Auges des Prüflings als die zweite Augeneigenschaft zu messen, oder ein optisches Hornhautformmesssystem, das daran angepasst ist, Messlicht auf eine Hornhaut des Auges des Prüflings zu projizieren und Reflexionslicht von der Hornhaut zu empfangen, um eine Form der Hornhaut als die zweite Augeneigenschaft zu messen.

## Revendications

1. Dispositif de mesure ultrasonore sans contact comprenant :
une première unité (10, 70) comportant un transducteur ultrasonore (10) adapté pour transmettre et recevoir une onde ultrasonore sans contact par rapport à un objet à examiner (E, S), la première unité étant adaptée pour traiter un signal de sortie du transducteur et mesurer une première caractéristique de l'objet, la première caractéristique étant la pression interne de l'objet ; et
une seconde unité (20, 60, 70, 100) comportant au moins une unité parmi une unité d'observation (20) pour observer l'objet sans contact à partir de l'avant et une unité de mesure (60, 100) pour mesurer une seconde caractéristique de l'objet sans contact à partir de l'avant,
dans lequel le transducteur comprend :
une ouverture (15) qui est pourvue dans la partie centrale du transducteur et qui a une taille suffisante pour être utilisée pour l'observation ou la mesure de l'objet par la seconde unité, et
une partie de transmission et de réception (11) de l'onde ultrasonore qui est pourvue pour entourer l'ouverture,
dans lequel
la partie de transmission et de réception (11) comporte une section de transmission (12) pour émettre un faisceau ultrasonore vers l'objet, et une section de réception (13) pour détecter le faisceau ultrasonore réfléchi par l'objet, et
caractérisé en ce que la première unité est configurée pour obtenir la pression interne de l'objet sur la base d'une relation entre l'amplitude du faisceau ultrasonore détecté par la section de réception et la pression interne de l'objet.

2. Dispositif de mesure ultrasonore sans contact selon la revendication 1, **caractérisé en ce que** l'unité d'observation ou l'unité de mesure comporte au moins une unité parmi une unité optique, une unité à rayons X, une unité ultrasonore, et une unité acoustique.

3. Dispositif de mesure ultrasonore sans contact selon l'une des revendications 1 à 2, **caractérisé en ce que** l'unité d'observation comporte une lentille d'objectif (22) à placer devant l'objet, et la lentille d'objectif est placée dans l'ouverture.

4. Dispositif de mesure ultrasonore sans contact selon l'une des revendications 1 à 3, **caractérisé en ce que** le transducteur ultrasonore est un transducteur à couplage par air à large bande pour transmettre et recevoir l'onde ultrasonore ayant une composante fréquentielle à large bande.

5. Dispositif de mesure ultrasonore sans contact selon l'une des revendications 1 à 4, **caractérisé en ce que** la partie de transmission et de réception est placée dans une forme presque annulaire à l'extérieur de l'ouverture.

6. Dispositif de mesure ultrasonore sans contact selon l'une des revendications 1 à 5, **caractérisé en ce qu'**une section de transmission et une section de réception de la partie de transmission et de réception sont agencées concentriquement dans des positions différentes.

7. Dispositif de mesure ultrasonore sans contact selon l'une des revendications 1 à 6, **caractérisé en ce que**
l'objet est l'oeil d'une personne examinée,
la première caractéristique est la pression intraoculaire, qui est une première caractéristique d'oeil de l'oeil de la personne examinée, et
l'unité d'observation est un système optique d'observation adapté pour observer un segment antérieur de l'oeil de la personne examinée à partir de l'avant.

8. Dispositif de mesure ultrasonore sans contact selon la revendication 7, **caractérisé en ce que**
la section de transmission est adaptée pour transmettre une onde d'impulsion ultrasonore vers le centre d'une cornée de l'oeil de la personne examinée et la section de réception est adaptée pour recevoir l'onde d'impulsion ultrasonore réfléchie par le centre de la cornée, et
la première unité de mesure obtient une valeur de pression intraoculaire sur la base d'une relation entre des caractéristiques d'onde de l'onde d'impulsion ultrasonore réfléchie détectée par la section de réception et la pression intraoculaire.

9. Dispositif de mesure ultrasonore sans contact selon la revendication 7 ou 8, **caractérisé en ce que**
l'unité de mesure est un système optique de mesure adapté pour mesurer une seconde caractéristique d'oeil de l'oeil de la personne examinée comme la seconde caractéristique à partir de l'avant, et l'ouverture entourée par la partie de transmission et de réception est utilisée comme un trajet optique de mesure du système optique de mesure.

10. Dispositif de mesure ultrasonore sans contact selon la revendication 9, **caractérisé en ce que** le système optique de mesure comporte au moins soit un système optique de mesure de pouvoir de réfraction de l'oeil adapté pour projeter une lumière de mesure sur un fond de l'oeil de la personne examinée et recevoir la lumière réfléchie du fond pour mesurer un pouvoir de réfraction d'oeil de l'oeil de la personne examinée comme la seconde caractéristique d'oeil soit un système optique de mesure de forme cornéenne adapté pour projeter une lumière de mesure sur une cornée de l'oeil de la personne examinée et recevoir la lumière de réflexion de la cornée pour mesurer une forme de la cornée comme la seconde caractéristique d'oeil.
